(19) Europäisches Patentamt
European Patent Office
Office européen des brevets

(11) EP 0 874 000 A2

# (12) EUROPÄISCHE PATENTANMELDUNG

(43) Veröffentlichungstag:
**28.10.1998 Patentblatt 1998/44**

(21) Anmeldenummer: **98105941.3**

(22) Anmeldetag: **01.04.1998**

(51) Int. Cl.$^6$: **C08B 37/00**, C08B 31/12, C08B 11/145, C08B 37/14, C02F 1/56, C02F 11/00, D21H 17/26, D21H 17/29, D21H 17/32, A61K 7/00, A61K 47/36

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **23.04.1997 DE 19717030**

(71) Anmelder:
**Degussa Aktiengesellschaft**
**60311 Frankfurt (DE)**

(72) Erfinder:
- **Fischer, Wolfgang**
  **63796 Kahl (DE)**
- **Brossmer, Christian, Dr.**
  **60318 Frankfurt (DE)**
- **Bischoff, Dietmar**
  **97340 Segnitz (DE)**
- **Rubo, Andreas, Dr.**
  **35440 Leihgestern (DE)**

## (54) Substituierte, pulverförmige, natürliche Polymere, ein Verfahren zur Herstellung und ihre Verwendung

(57) Die Erfindung betrifft hochsubstituierte, pulverförmige natürliche Polymere wie z. B. Stärke, Cellulose oder Galaktomannane, mit Substitutionsgraden (DS: degree of substitution) >0,32, ein Verfahren zur Herstellung dieser in Pulverform vorliegenden Verbindungen mit Hilfe einer mehrstufig durchzuführenden Umsetzung ohne Vorverkleisterung und die Verwendung als Flockungshilfsmittel für wässrige Suspensionen und Abwässer.

EP 0 874 000 A2

## Beschreibung

Die Erfindung betrifft hochsubstituierte, pulverförmige natürliche Polymere wie z. B. Stärke, Cellulose oder Galaktomannane, mit Substitutionsgraden (DS: degree of substitution) >0,32, ein Verfahren zur Herstellung dieser in Pulverform vorliegenden Verbindungen mit Hilfe einer mehrstufig durchzuführenden Umsetzung und die Verwendung.

Sehr hoch kationisierte Stärken mit einem Substitutionsgrad (DS) größer 0,30 sind schon seit längerem, wenn auch in gelöster Form bekannt und werden unter anderem als Flockungshilfsmittel für wässrige Suspensionen und Abwässer negativ geladener organischer oder anorganischer Inhaltsstoffe wie z.B. Ton, Titandioxid, Kohle, Eisenerz, anionischer Stärke und Cellulose eingesetzt. Eine Übersicht der Anwendungen gelöster hochkationisierter Stärken ist in "Modified Starches: Properties and Uses" von O.B. Wurzburg, CRC Press, 1986, zu finden.

Die Herstellung dieser sehr hoch kationisierten Stärkeprodukte wird in den US-PS 2,995,513 und 3,842,005 sowie in der US-PS 3,835,114 beschrieben und erfolgt durch Umsetzung nativer, konvertierter oder viskositätsregulierter Stärken im gelatinisierten Zustand mit quartären, kationischen Verätherungsmitteln. Die vorliegende simultane Verkleisterung und Reaktion der Stärke mit den Kationisierungsreagenzien wird in der Fachsprache als „in situ"- oder Kleister-Kationisierung bezeichnet.

Der US-PS-3,842,005 kann entnommen werden, daß kationische Stärkeether mit einem DS > 0,7 erhalten werden, wenn native Stärke in wäßriger Suspension bei höherer Temperatur verkocht und gleichzeitig mit dem Kationisierungsreagenz 3-Chlor-2-hydroxypropyltrimethylammoniumchlorid in Gegenwart eines alkalischen Katalysators veräthert wird. Die US-PS-2,995,513 betrifft ein ähnliches Verfahren, wonach kationische Stärken mit einem Substitutionsgrad von 0,18 - 0,66 durch Verkochung und Reaktion mit 2,3-Epoxypropyltrimethylammoniumchlorid erhalten werden. Die so erhaltenen kationischen Stärkeprodukte mit sehr hohem Kationisierungsgrad (DS >0,3) können nach einem Reinigungsschritt, der eine Dialyse, Ultrafiltration oder einen Ionenaustausch beinhaltet, als Flockungshilfsmittel eingesetzt werden (US-PS-3,842,005).

Ein Nachteil dieser durch „in situ""-Kationisierung hergestellten Stärken mit einem DS größer 0,3 ist der hohe Energieaufwand zum Verkochen der Stärke und die geringen Kationisierungsausbeuten, welche durch Nebenreaktionen des Verätherungsreagenzes beim Verkochen (T > 70 °C) im wässrigen Medium in Kauf genommen werden müssen. Dies hat zusätzlich zur Folge, daß die im verkleisterten Zustand erhaltenen kationischen Stärken in hohem Maße mit Nebenprodukten verunreinigt sind und durch die oben aufgeführten Trennoperationen aufgereinigt werden müssen.

Wirtschaftlich interessant wäre es, ein pulverförmiges, hochsubstituiertes Polymer herzustellen ohne daß eine Vorverkleisterung stattfindet, und dieses dem jeweiligen Anwender, z. B. einer Kläranlage, zu liefern.

Die Begründung dafür liegt darin, daß diese Anwender keine Vorrichtungen besitzen, um die hochsubstituierten Polymere selbst nach dem Stand der Technik durch Verkochen herzustellen, und dies aufgrund der geringen Ausbeuten auch nicht erstrebenswert ist.

Um ein pulverförmiges, trockenes Produkt zu erhalten, müßten die „in-situ" kationisierten Stärken in zusätzlichen Prozessen durch Zugabe von Lösungsmitteln ausgefällt und anschließend getrocknet werden (s. US-PS 2,995,513, Spalte 5, Zeile 1-7). Aufgrund dieser Nachteile erweist sich das „in situ"-Kationisierungsverfahren für die Herstellung hoch-kationisierter Stärken mit einem DS größer 0,3 gegenwärtig als nicht wirtschaftlich.

Auch das kommerziell gängige Kationisierungsverfahren in wäßriger Suspension mit 35 - 45 % Feststoffanteil, das sogenannte „Slurry-Verfahren", welches bei einer Temperatur unterhalb der Verkleisterungstemperatur der Stärke arbeitet, ist keine Alternative. Hier wird die Stärke nach der Umsetzung mit dem kationischen Verätherungsmittel zwar in granulärer Form zurückerhalten, aber sind mit diesem Verfahren in wässriger Lösung keine Substitutionsgrade > 0,3 erreichbar. Dies ist darauf zurückzuführen, daß die Verkleisterungstemperatur der Stärke mit zunehmendem Substitutionsgrad absinkt und kationische Stärken ab einem Substitutionsgrad von etwa 0,07 zunehmend kaltwasserlöslich werden. Bei Substitutionsgraden größer 0,3 sind die Produkte als kationische Stärkekleister in Wasser gelöst. Eine Herstellung solch hoch kationisierter Stärken in einem rein wässrigen Slurry-Prozeß würde somit die gleichen Aufarbeitungsschritte erfordern wie das oben genannte „in-situ"-Verfahren.

Aus dem Stand der Technik sind jedoch auch Verfahren bekannt, die z.B. zu einer pulverförmigen kationisierten Stärke führen.

In der DE-OS- 29 35 338 wird ein Verfahren zur Herstellung einer kationisierten Stärke mit einem "Stickstoffsubstituierungsgrad" von 0,95 bis höchstens 2,8 % beschrieben. Unter Stickstoffsubstituierungsgrad versteht man den polymergebundenen, kationischen Stickstoffgehalt pro 100 g absolut trockener Stärke, d.h. g $N_{kation}$/100 g atro Stärke, so daß dies einem theoretischen Substitutionsgrad von DS = 0,10 - 0,32 entspricht.

Mit der EP 0 303 039 B1 ist ein ähnliches Verfahren bekannt, das trotz niedriger Reaktionstemperaturen gleichzeitig sehr wenig Rührenergie benötigt, Reaktionsgefäße nur kurzzeitig blockiert und gute Ausbeuten liefert. Die Umsetzung der Stärke mit dem Verätherungsreagenz erfolgt bei 5 bis 40°C in Gegenwart einer feinteiligen, hydrophilen Kieselsäure und Alkalisilikaten und/oder -aluminaten oder eines Gemisches aus Alkali- oder Erdalkalihydroxiden bzw. -oxiden und Alkalicarbonaten oder eines Gemisches dieser Hydroxide, Oxide oder Carbonate mit einem Alkalisilikat und/oder - aluminat. Das die Stärke enthaltende Reaktionsgemisch wird in einem Intensivmischer innerhalb von 10

Sekunden bis 25 Minuten homogenisiert, ausgeschleust und in einem hierfür vorgesehenen Lagerbehältnis zum vollständigen Ausreagieren gebracht.

Allen oben aufgeführten Trockenverfahren ist gemeinsam, daß die hierbei erzielten, praktischen Substitutionsgrade ($DS_{prakt.}$) bei DS $\leq$ 0,32 liegen und die Umsetzungen in einem einzigen Reaktionsschritt durchgeführt werden. Der praktisch erreichte Substitutionsgrad ($DS_{prakt.}$) umfaßt nur den an das Polymer chemisch gebundenen Anteil des Verätherungsreagenzes. Er wird mit Hilfe einer Stickstoffbestimmung nach Kjeldahl über den kationisch gebundenen Stickstoff ($\%N_{kation.}$) analytisch erfaßt und bei der Verwendung von 2,3-Epoxypropyltrimethylammoniumchlorid bzw. 3-Chlor-(2-hydroxypropyl)trimethylammoniumchlorid als Verätherungsreagenzien nach folgender Formel berechnet:

$$DS_{(prakt.)} = \frac{\%\,N_{kation.} * 162,15}{1401 - (\%N_{kation.} * 151,64)}$$

(Der gebundene kationische Stickstoffgehalt ($\%N_{kation}$) ergibt sich als Differenz zweier Stickstoffbestimmungen und zwar durch Abziehen des unter den Kationisierungsbedingungen unlöslichen Proteinstickstoffs ($\%N_{unlösl.}$) von dem nach dem Auswaschen bestimmten Stickstoffgehalt

$$(\%N_{ausgew.}):\ \%N_{kation.} = \%N_{ausgew.} - \%N_{unlösl.})$$

Nach Umstellung dieser Formel und Einsetzen eines Substitutionsgrades von $DS_{(prakt.)} \leq 0,32$ ergibt sich die äquivalente Aussage, daß bei allen oben aufgeführten, bekannten Trockenverfahren der polymergebundene kationische Stickstoffgehalt ($\%N_{kation}$) bei $\leq$ 2,1 % liegt.

Für den Einsatz als umweltfreundliches Flockungshilfsmittel und Schlammentwässerungsmittel bei der Abwasserbehandlung sowie für den Gebrauch als Verdickungsmittel, Retentionshilfsmittel und Störstoffänger bei der Papierherstellung erweisen sich zunehmend sehr hoch kationisierte natürliche Polymere als geeignete Materialien. Es besteht daher ein Bedürfnis, diese bislang nur durch „in-situ"-bzw. Kleister-Kationisierung zugänglichen Polymere in einem ausbeutestarken, prozeßtechnisch einfachen und energetisch günstig arbeitenden Verfahren bereitzustellen.

Gegenstand der Erfindung sind nicht vorverkleisterte, pulverförmige, substituierte natürliche Polymere, die über Ether- oder Iminogruppen gebundene Aminverbindungen der allgemeinen Formeln:

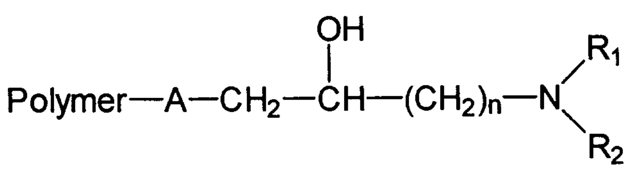

(I)

oder

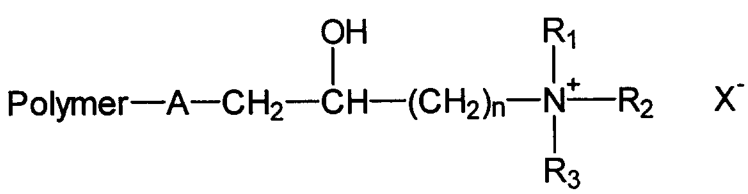

(II)

in der n eine ganze Zahl von 1 bis 3 ist, $R_1$,$R_2$ und $R_3$ gleiche oder verschiedene Alkylreste mit 1 bis 20 Kohlenstoffatomen insbesondere 1 bis 4 C-Atome, bevorzugt Methyl, Äthyl oder $R_1$ den Benzylrest darstellt, und $X^-$ Chlorid, Bromid, Sulfat, Nitrat oder Acetat bedeuten, insbesondere aber Chlorid, enthalten, wobei der Substitutionsgrad (D.S.)bei >0,32,insbesondere bis $\leq$ 3 bevorzugt bei 0,4 bis 2, besonders bei 0,4 bis 0,8 liegt.

Wenn einer der Reste $R_1$, $R_2$ oder $R_3$ einen Alkylrest mit mehr als 10 C-Atomen darstellt, entsprechen die anderen bevorzugt Resten mit kürzerer C-Kette.

A bedeutet Sauerstoff-O- oder Imino-NH-, bevorzugt aber Sauerstoff. Je nach Proteingehalt der substituierten Stärke liegen auch beide Bedeutungen nebeneinander vor.

Pulverförmig bedeutet in diesem Zusammenhang, daß es sich um ein trotz seines Wassergehalts als trocken erscheinendes Pulver mit einer zufriedenstellenden Rieselfähigkeit handelt.
Pulverförmig bedeutet hier auch, daß die Produkte in einer nicht vorverkleisterten, d. h. nichtgelatinisierten, Form vorliegen.
Letzteres ist wegen der Dosierbarkeit bei seiner Anwendung von Bedeutung. Der Wassergehalt des Produkts liegt bevorzugt zwischen 10 und 35 Gew.-%.

Rohstoff für die erfindungsgemäßen neuen Stoffe sind native oder modifizierte natürliche Polymere oder natürliche Polymere enthaltende Substanzen beliebiger Herkunft. Mit besonderem Vorteil werden native Stärken wie Weizen-, Mais-, Kartoffel- und Tapiokastärke sowie B-Stärken mit hohen Proteingehalten verwendet. Andere natürliche Polymere wie Guarkernmehl, Cellulose und Proteine können in gleicher Weise substituiert werden.

In einer bevorzugten Ausführungsform enthält das substituierte Polymer eine feinteilige Kieselsäure in einer Menge von 0,02 bis 2,0 Gew.-%.
Dabei handelt es sich um gefällte oder durch Flammenhydrolyse erzeugte Kieselsäuren, hydrophil oder hydrophob.
Die spezifischen Oberflächen liegen zwischen 60 und 700 $m^2/g$, bevorzugt 100 und 450 $m^2/g$ (BET-Messung nach DIN 66 131, $N_2$-Adsorption bei der Temperatur des flüssigen Stickstoffs, vorheriges Ausheizen der Probe bei 100 °C).
Bevorzugt eingesetzt werden hydrophile gefällte Kieselsäuren mit einer spezifischen Oberfläche von 190 bis 450 $m^2/g$, insbesondere eine sprühgetrocknete gefällte Kieselsäure mit einer spezifischen Oberfläche von 190 $m^2/g$ (BET-Messung).
Man verwendet auch Gemische aus hydrophoben und hydrophilen Kieselsäuren.

Der pH-Wert der erfindungsgemäßen Verbindungen ist nicht festgelegt.
Wird keine Neutralisation vorgenommen, stellt sich bei einer 1 Gew.-%igen Lösung (Kleister) ein pH-Wert von bevorzugt > 8 ein.

Man kann dem Reaktionsprodukt jedoch auch im Laufe oder nach der Umsetzung mit den Alkylenepoxiden anorganische Säuren, wie Salzsäure, Salpetersäure oder Phosphorsäure oder auch bevorzugt Citronensäure oder Adipinsäure zusetzen.
Man erhält so Produkte mit einem pH-Wert (1 Gew.-%ige Lösung (Kleister) von ~6, bevorzugt von 5 bis 7.

Im Gegensatz zu den nach dem Stand der Technik bekannten Produkten wird hiermit zum ersten Mal ein hochsubstituiertes Polymer als Pulver zur Verfügung gestellt.

Gegenstand der Erfindung ist ebenso ein Verfahren zur Herstellung von substituierten natürlichen Polymeren mit einem Substitutionsgrad > 0,32, das dadurch gekennzeichnet ist, daß man die benötigte Gesamtmenge der Alkylenepoxide der allgemeinen Formeln

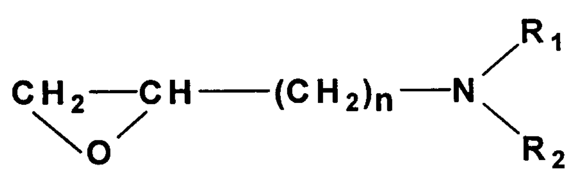

oder

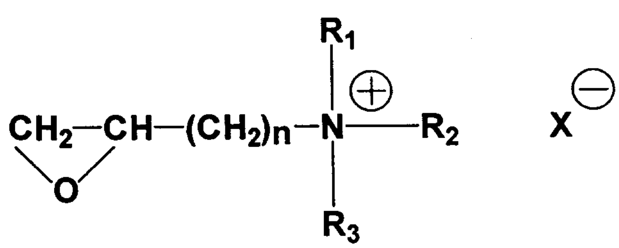

in denen n, $R_1$, $R_2$, $R_3$ dieselbe Bedeutung wie oben haben, in mindestens zwei Teilmengen aufteilt und diese in Gegenwart einer alkalisch wirkenden anorganischen Verbindung, 10 bis 35 Gew.-% Wasser (bezogen auf atro Stärke) und 0,02 bis 2 Gew.-% einer feinteiligen hydrophoben oder hydrophilen Kieselsäure (bezogen auf das Reaktionsgemisch) mit dem Polymeren stufenweise umsetzt.

Die erfindungsgemäße Umsetzung erfolgt in einem Temperaturbereich von 5 bis 90 ° C, bevorzugt 20 bis 60 ° C.
Zusätzlich erweist es sich als vorteilhaft, wenn man in einem Temperaturbereich arbeitet, in dem sich in Abhängigkeit von z. B. jahreszeitlich bedingten Schwankungen der Umgebungstemperatur die Raumtemperatur von anspruchslosen Warenlagern bewegt, d. h. von 18 bis 30 ° C, insbesondere 20 bis 25 ° C.

Nach dem Vermischen der Reaktanden wartet man die vollständige Umsetzung bevorzugt in dem Lagerbehältnis

ab, in das die Reaktionsmischung abgefüllt wird.
Letzteres gilt vor allem für die letzte Stufe des erfindungsgemäßen Verfahrens.
Vorzugsweise setzt man in der 1. Stufe und jeder weiteren Stufe je Mol Polymer, (berechnet als Anhydroglucoseeinheit) 0,1 bis 0,3 Mol, bevorzugt 0,18 bis 0,25 Mol eines der obengenannten Alkylenepoxide zu, bis der gewünschte DS-Wert erreicht ist.
Daraus folgt im allgemeinen ein praktischer Substitutionsgrad (D.S.prakt.) von jeweils 0,07 bis 0,27, bzw. von 0,14 bis 0,22.
Um die Umsetzungsdauer in den aufeinanderfolgenden Stufen abzukürzen, arbeitet man in der 1. und den weiteren Stufen vor der letzten Stufe bevorzugt bei Temperaturen von 25 bis 60 ° C.
Man arbeitet in mindestens zwei und bis zu sechs Stufen, vorzugsweise 3 bis 5 Stufen, um DS-Werte von 0,33 bis 0,85, bevorzugt 0,40 bis 0,70 zu erreichen.

Als alkalisch wirkende Verbindungen setzt man insbesondere Alkalisilikate und/oder -aluminate oder Alkali- oder Erdalkalihydroxide bzw. -oxide oder Alkalicarbonate oder ein Gemisch einer oder mehrerer dieser Hydroxide Oxide oder Carbonate mit einem Alkalisilikat und/oder -aluminat ein.

Alkali bedeutet insbesondere Na oder K, Erdalkali insbesondere Ca.
Sie werden in Abhängigkeit von der zur Modifizierung der Polymere eingesetzten Menge an Alkylenepoxiden in entsprechenden Stufen dosiert.

Dabei wurde gefunden, daß man bei Verwendung bestimmter alkalisch wirkender Verbindungen (Aktivatoren) die Viskosität der aus hochkationisierten Polymeren herstellbaren Lösungen nach Wunsch einstellen kann.

Durch Variation der Gehalte an Erdalkalihydroxiden und Alkalisilikaten und/oder -aluminaten in den Aktivatoren lassen sich bei konstantem Substitutionsgrad des Polymeren die Viskositäten (Brookfield) in wässriger Lösung im Bereich von 50 mPas bis 5000 mPas, bevorzugt von 100 mPas - 2000 mPaS, einstellen.
So bewirkt z. B. eine Erhöhung des Natriummetasilikatanteils im Aktivator eine Zunahme der Brookfield-Viskosität (s. Beispiel 3).

Bevorzugt setzt man die alkalisch wirkenden Verbindungen in Form der aus den EP-B 0233 336 und EP-B 0303 039 bekannten Abmischungen mit feinteiliger Kieselsäure (Aktivatoren) ein.

Erfindungsgemäß geht man dabei so vor, daß man die Mischung aus Stärke, den Teilmengen der alkalisch wirkenden Verbindungen,der Kieselsäure und des Alkylenepoxids, das im allgemeinen als wässrige Lösung eingesetzt wird, in einem Intensivmischer jeweils innerhalb von 10 Sekunden bis 25 Minuten, bevorzugt 20 Sekunden bis 5 Minuten, homogenisiert, sie dann ausschleust und bevorzugt bei einer Temperatur von 25 bis 60 ° C ausreagieren läßt.

Dieser Vorgang wird dann im allgemeinen wiederholt, bis der gewünschte DS-Wert erreicht ist.
Bevorzugt setzt man die alkalisch wirkende Verbindung allein oder mit der Kieselsäure vermischt (Aktivator) dem Polymer vor dem Alkylenepoxid zu.
Die letzte Stufe der Umsetzung läßt man bevorzugt in dem vorgesehenen Lagerbehältnis, wie z. B. einem Silo oder auch in den für den Versand vorgesehenen Verpackungen, bis zum Ende ablaufen, ohne daß hier eine Temperaturerhöhung notwendig ist.
Weitere Mischenergien müssen nicht für zusätzliche Verfahrenschritte aufgewendet werden.
Es ist auch möglich, statt der Alkylenepoxide die entsprechenden Vorstufen dieser Verbindungen, die Chlorhydrine, zu verwenden.
Dies ist jedoch bei der Bemessung der alkalisch wirkenden Verbindungen zu berücksichtigen, da eine bekannte Menge zur Umwandlung in die reaktiven Epoxide benötigt wird.

Das erfindungsgemäße Verfahren ist kontinuierlich und diskontinuierlich durchführbar.

Als Intensivmischer setzt man bevorzugt Pflugscharmischer oder insbesondere bei kontinuierlicher Durchführung, Durchflußbefeuchtungsmischer ein.

Man erhält erstmals ein hochsubstituiertes Polymer auf Naturbasis in trockener, gut handhabbarer Form, das frei von Klumpen ist und nicht mehr gesiebt werden muß.

Dieses Produkt ist außerdem weitestgehend frei von unerwünschten Nebenprodukten.

Wenn es gewünscht wird, kann im Anschluß an die Umsetzung trotzdem eine Vermahlung, Siebung sowie eine Neutralisation des Polymers erfolgen.

Für die Neutralisation des nach dem beschriebenen Verfahren hergestellten substituierten Polymers wird eine entsprechend der alkalischen Komponente notwendige Menge Säure zugemischt. Als vorteilhaft hat sich hierbei die Verwendung von Adipinsäure erwiesen. Es können jedoch auch andere organische oder anorganische Säuren sowie saure Salze, z.B. Zitronensäure, Fumarsäure, Amidoschwefelsäure, Salzsäure, Aluminiumsulfat und Aluminiumchlorid für die Neutralisation verwendet werden.

Das hochsubstituierte Polymer kann auch mit einem geeigneten Lösungsmittel bzw. wässrigen Lösungsmittelgemisch (z.B. 75% Isopropanol in Wasser ) gewaschen und anschließend getrocknet werden.

Die erfindungsgemäßen kationischen Polymere mit einem Substitutionsgrad > 0,32 eignen sich besonders als Flockungshilfsmittel bei der Abwasserbehandlung (z.B. in Kläranlagen ). Darüberhinaus eignen sie sich als Schlammentwässerungsmittel, Verdickungsmittel, Retentionshilfsmittel sowie als Störstoffänger bei der Papierherstellung.Die nach dem beschriebenen Verfahren hergestellten und gereinigten hochkationischen natürlichen Polymere können zusätzlich in der Kosmetik- und Pharmaindustrie eingesetzt werden.

## Beispiele

Die nachfolgenden Beispiele illustrieren den Gegenstand der Erfindung.

Durch intensives Vermischen werden folgende Aktivator-Typen für die Kationisierung von natürlichen Polymeren hergestellt:

| Type | Zusammensetzung (Gew.-%) |
|---|---|
| PC-3 | 75 % Calciumhydroxid, ca. 98 %ig |
| | 25 % Kieselsäure (sprühgetrocknete, gefällte Kieselsäure, 190 $m^2/g$ BET) |
| PC-4 | 40 % Calciumhydroxid, ca. 98 %ig |
| | 40 % Natriummetasilikat, wasserfrei |
| | 20 % Kieselsäure |
| PC-5 | 8,5 % Calciumhydroxid, ca. 98 %ig |
| | 76,5 % Natriummetasilikat, wasserfrei |
| | 15,0 % Kieselsäure |

Beispiel 1

Herstellung einer kationischen Kartoffelstärke mit einem Substitutionsgrad von DS = 0,82
(Kationisierung in 5 Stufen)

In einem 50 l Pflugscharmischer mit Messerkopf (Typ FM 50 , Fa. Lödige) wird für die erste Stufe 10,0 kg native Kartoffelstärke (Feuchtegehalt 18,0 %, Gehalt an unlöslichem Stickstoff 0,010%) 5 Minuten lang intensiv mit 0,246 kg Aktivator PC-3 vermischt. Anschließend wird eine Reagenzlösung (Quab-Wasser-Mischung), welche 1,534 kg 2,3-Epoxypropyltrimethylammoniumchlorid enthält (entsprechend einem theoretischen DS von 0,2), bei laufendem Mischer innerhalb von 2 Minuten aufgesprüht. Nach einer weiteren Mischdauer von 5 Minuten (Nachmischzeit) wird das Reaktionsgut in Polyethylensäcken verpackt und 1 Tag bei 50 °C gelagert.

Die Durchführung der folgenden Stufen erfolgt in ähnlicher Weise. Das kationisierte Produkt der jeweils vorangegangenen Stufe wird zuerst mit der jeweils angebenenen Menge Aktivator PC-3 und einer zusätzlich benötigten Menge sprühgetrockneter, gefällter Kieselsäure 5 Minuten lang intensiv vermischt. Anschließend wird in analoger Weise eine Reagenzlösung (Quab- Wasser-Mischung), die 1,534 kg 2,3-Epoxypropyltrimethylammoniumchlorid enthält, bei laufendem Mischer innerhalb von 2 Minuten aufgesprüht. Nach einer weiteren Mischdauer von 5 Minuten wird das Reaktionsgut wieder in Polyethylensäcken verpackt und 1 Tag bei 50 °C gelagert.

Die Ausbeute bzw. der Substitutionsgrad (DS) der einzelnen Stufen wird jeweils durch Stickstoffbestimmung nach Kjeldahl am ausgewaschenen Produkt bestimmt.

Im folgenden sind die jeweiligen Rezepturen und Ausbeuten bzw. Substitutionsgrade der einzelnen Reaktionsstufen aufgeführt:

**<u>1. Stufe ( Theor. Substitutionsgrad = 0,20 )</u>**

| | |
|---|---|
| 10,000 kg | native Kartoffelstärke, 18% Feuchte, = 8,200 kg atro = 0,05057 kMol |
| + 0,246 kg | Aktivator PC-3 = 3,0% bez. auf atro Stärke |
| + 2,087 kg | QUAB 151 ( 73,5% Ges.Epoxid, 21,4% Wasser ) = 0,01012 kMol |
| <u>+ 0,529 kg</u> | <u>Wasser</u> |
| = 12,862 kg | Gesamtansatz (21,6% Feuchte) |

Mischzeit / Aktivator = 5 Minuten

QUAB / Wasser-Zugabe = 2 Minuten
Nachmischzeit = 5 Minuten

Reaktionszeit : 1 Tag / 50°C
Analyse : 1,32% Stickstoff (gewaschen, atro)
Substitutionsgrad ( DS ) = 0,179 ⇒ Ausbeute (1.Stufe) = 89,4 %

**2. Stufe ( Theor. Substitutionsgrad = 0,20 + 0,20 = 0,40 )**

| | |
|---|---|
| 12,862 kg | kationische Kartoffelstärke (1.Stufe) DS 0,179, 21,6% Feuchte, = 8,200 kg atro Stärke = 0,05057 kMol |
| + 0,123 kg | Aktivator PC-3 = 1,5% bez. auf atro Stärke |
| + 0,082 kg | Kieselsäure = 1,0% bez. auf atro Stärke |
| + 2,087 kg | QUAB 151 (73,5% Ges.Epoxid, 21,4% Wasser) = 0,01012 kMol |
| + 0,016 kg | Wasser |
| =15,170 kg | Gesamtansatz (21,5% Feuchte) |

Mischzeit / Aktivator = 5 Minuten
QUAB / Wasser-Zugabe = 2 Minuten
Nachmischzeit = 5 Minuten

Reaktionszeit : 1 Tag / 50°C
Analyse : 2,29% Stickstoff (gewaschen, atro)
Substitutionsgrad (DS) = 0,353 ⇒ Ausbeute (1.+2.Stufe) = 88,2 %
ΔDS (2.-1.Stufe) = 0,353 - 0,179 = 0,174 ⇒ Ausbeute (2 Stufe) = 87,0%

**Gesamt-Ansatzmengen ( 2 Stufen ) Theor.DS = 0,40**

| | |
|---|---|
| 10,000 kg | native Kartoffelstärke, 18 % Feuchte |
| 0,369 kg | Aktivator PC-3 |
| 0,082 kg | Kieselsäure |
| 4,174 kg | QUAB 151, 73,5% Ges.Epoxid |
| 0,545 kg | Wasser |
| 15,170 kg | |

**3. Stufe (Theor. Substitutionsgrad = 0,20 + 0,20 + 0,20 = 0,60)**

| | |
|---|---|
| 15,170 kg | kationische Kartoffelstärke (2.Stufe) DS 0,353, 21,5% Feuchte 8,200 kg atro Stärke = 0,05057 kMol |
| + 0,123 kg | Aktivator = PC-3 = 1,5% bez. auf atro Stärke |
| + 0,082 kg | Kieselsäure = 1,0% bez. auf atro Stärke |
| + 2,087 kg | QUAB 151 ( 73,5% Ges.Epoxid, 21,4% Wasser) = 0,01012 kMol |
| + 0,016 kg | Wasser |
| =17,478 kg | Gesamtansatz (21,3% Feuchte) |

Mischzeit / Aktivator = 5 Minuten
QUAB / Wasser-Zugabe = 2 Minuten
Nachmischzeit = 5 Minuten

Reaktionszeit : 1 Tag / 50°C
Analyse : 3,04% Stickstoff ( gewaschen, atro)
Substitutionsgrad ( DS ) = 0,525 ⇒ Ausbeute (1. - 3. Stufe) = 87,5 %
ΔDS (3.-2.Stufe) = 0,525 - 0,353 = 0,172 ⇒ Ausbeute (3.Stufe) = 86,0 %

Gesamt-Ansatzmengen ( 3 Stufen ) Theor.DS = 0,60

| | |
|---|---|
| 10,000 kg | native Kartoffelstärke, 18% Feuchte |
| 0,492 kg | Aktivator PC-3 |
| 0,164 kg | Kieselsäure |
| 6,261 kg | QUAB 151, 73,5% Ges.Epoxid |

<u>0,561 kg</u> <u>Wasser</u>
17,474 kg

**<u>4. Stufe ( Theor. Substitutionsgrad = 0,20 + 0,20 + 0,20 + 0,20 = 0,80)</u>**

17,478 kg kationische Kartoffelstärke (3.Stufe) DS 0,525, 21,3% Feuchte = 8,200 kg atro Stärke = 0,05057 kMol
+ 0,123 kg Aktivator PC-3 = 1,5% bez. auf atro Stärke
+ 0,082 kg Kieselsäure = 1,0% bez. auf atro Stärke
+ 2,087 kg QUAB 151 (73,5% Ges.Epoxid, 21,4% Wasser) = 0,01012 kMol <u>+ 0,016 kg Wasser</u>
=19,786 kg Gesamtansatz (21,2% Feuchte)

Mischzeit / Aktivator = 5 Minuten
QUAB / Wasser-Zugabe = 2 Minuten
Nachmischzeit = 5 Minuten

Reaktionszeit : 1 Tag / 50°C
Analyse : 3,57% Stickstoff ( gewaschen, atro )
Substitutionsgrad ( DS ) = 0,675 ⇒ Ausbeute (1. - 4.Stufe) = 84,4 %
ΔDS (4.-3. Stufe) = 0,675 - 0,525 = 0,150 ⇒ Ausbeute (4.Stufe) = 75,0 %

**<u>Gesamt-Ansatzmengen ( 4 Stufen ) Theor.DS = 0,80</u>**

10,000 kg native Kartoffelstärke, 18% Feuchte
0,615 kg Aktivator PC-3
0,246 kg Kieselsäure
8,348 kg QUAB 151, 73,5% Ges.Epoxid
<u>0,577 kg</u> <u>Wasser</u>
19,786 kg

**<u>5. Stufe ( Theor. Substitutionsgrad = 0,20 + 0,20 + 0,20 + 0,20 + 0,20 = 1,00)</u>**

19,786 kg kationische Kartoffelstärke (4.Stufe) DS 0,6925, 21,2% Feuchte = 8,200 kg atro Stärke = 0,05057 kMol

+ 0,123 kg Aktivator PC-3 = 1,5% bez. auf atro Stärke
+ 0,082 kg Kieselsäure = 1,0% bez. auf atro Stärke
+ 2,087 kg QUAB 151 (73,5% Ges.Epoxid, 21,4% Wasser) = 0,01012 kMol
<u>+ 0,016 kg</u> <u>Wasser</u>
22,094 kg Gesamtansatz (21,1% Feuchte)

Mischzeit / Aktivator = 5 Minuten
QUAB / Wasser-Zugabe = 2 Minuten
Nachmischzeit = 5 Minuten

Reaktionszeit : 1 Tag / 50°C
Analyse : 4,00 % Stickstoff ( gewaschen, atro )
Substitutionsgrad ( DS ) = 0,816 ⇒ Ausbeute (1. - 5.Stufe) = 81,6 %
ΔDS (5.-4.Stufe) = 0,816 - 0,675 = 0,141 ⇒ Ausbeute (5.Stufe) = 70,5 %

<u>Gesamt-Ansatzmengen ( 5 Stufen ) Theor.DS = 1,00</u>

10,000 kg native Kartoffelstärke, 18% Feuchte
0,738 kg Aktivator PC-3
0,328 kg Kieselsäure
10,435 kg QUAB 151, 73,5% Ges.Epoxid
<u>0,593 kg</u> <u>Wasser</u>
22,094 kg

Vergleichsbeispiel 1a

Herstellung einer hochkationischen Kartoffelstärke gemäß EP-A 0 303 039 (Degussa) mit einem theoretischen Substitutionsgrad von DS = 0,40

Es wurde wie in Beispiel 1 verfahren, jedoch mit dem Unterschied, daß die Kationisierung in einer Stufe durchgeführt wurde und die Ansatzmengen einem theoretischen Substitutionsgrad von 0,40 entsprechen.

Hierzu wurden in einem 50 l Pflugscharmischer mit Messerkopf (Typ FM 50 , Fa. Lödige) 10,0 kg native Kartoffelstärke (Feuchtegehalt 18,0 %, Gehalt an unlöslichem Stickstoff 0,010%) 5 Minuten lang intensiv mit 0,369 kg Aktivator PC-3 und 0,082 kg sprühgetrockneter Kieselsäure vermischt. Anschließend wird eine Reagenzlösung (Quab-Wasser-Mischung), welche 3,068 kg 2,3-Epoxypropyltrimethylammoniumchlorid enthält, (entsprechend einem theoretischen DS von 0,40) bei laufendem Mischer innerhalb von 2 Minuten aufgesprüht. Nach einer weiteren Mischdauer von 5 Minuten (Nachmischzeit) wird das Reaktionsgut in Polyethylensäcken verpackt und 1 Tag bei 50 °C gelagert.

Analyse: 1,93 % Stickstoff (gewaschen, atro)

Substitutionsgrad (DS) = 0,283 $\Rightarrow$ Ausbeute = 70,8 %

Vergleichsbeispiel 1 b

Herstellung einer hochkationischen Kartoffelstärke gemäß EP-A 0 303 039 (Degussa) mit einem theoretischen Substitutionsgrad von DS = 0,60

Es wurde wie in Beispiel 1 verfahren, jedoch mit dem Unterschied, daß die Kationisierung in einer Stufe durchgeführt wurde und die Ansatzmengen einem theoretischen Substitutionsgrad von 0,60 entsprechen.

Hierzu wurden in einem 50 l Pflugscharmischer mit Messerkopf (Typ FM 50 , Fa. Lödige) 10,0 kg native Kartoffelstärke (Feuchtegehalt 18,0 %, Gehalt an unlöslichem Stickstoff 0,010%) 5 Minuten lang intensiv mit 0,492 kg Aktivator PC-3 und 0,164 kg sprühgetrockneter Kieselsäure vermischt. Anschließend wird eine Reagenzlösung (Quab-Wasser-Mischung), welche 4,602 kg 2,3-Epoxypropyltrimethylammoniumchlorid enthält, (entsprechend einem theoretischen DS von 0,60) bei laufendem Mischer innerhalb von 2 Minuten aufgesprüht. Nach einer weiteren Mischdauer von 5 Minuten (Nachmischzeit) wird das Reaktionsgut in Polyethylensäcken verpackt und 1 Tag bei 50 °C gelagert.

Analyse: 2,13 % Stickstoff (gewaschen, atro)
Substitutionsgrad (DS) = 0,320 $\Rightarrow$ Ausbeute = 53,4 %

Vergleichsbeispiel 1c

Herstellung einer hochkationischen Kartoffelstärke gemäß EP-A 0 303 039 (Degussa) mit einem theoretischen Substitutionsgrad von DS = 1,00

Es wurde wie in Beispiel 1 verfahren, jedoch mit dem Unterschied, daß die Kationisierung in einer Stufe durchgeführt wurde und die Ansatzmengen einem theoretischen Substitutionsgrad von 1,00 entsprechen.

Hierzu wurden in einem 50 l Pflugscharmischer mit Messerkopf (Typ FM 50 , Fa. Lödige) 10,0 kg native Kartoffelstärke (Feuchtegehalt 18,0 %, Gehalt an unlöslichem Stickstoff 0,010%) 5 Minuten lang intensiv mit 0,738 kg Aktivator PC-3 und 0,328 kg sprühgetrockneter Kieselsäure vermischt. Anschließend wird eine Reagenzlösung (Quab-Wasser-Mischung), welche 7,670 kg 2,3-Epoxypropyltrimethylammoniumchlorid enthält, (entsprechend einem theoretischen DS von 1,00) bei laufendem Mischer innerhalb von 2 Minuten aufgesprüht. Nach einer weiteren Mischdauer von 5 Minuten (Nachmischzeit) wird das Reaktionsgut in Polyethylensäcken verpackt und 1 Tag bei 50 °C gelagert.

Analyse: 2,07 % Stickstoff (gewaschen, atro)

Substitutionsgrad (DS) = 0,309 $\Rightarrow$ Ausbeute = 30,9 %

Die Vergleichsbeispiele 1a-c zeigen, daß ein praktischer Substitutionsgrad von DS > 0,32 in einer einzigen Reaktionsstufe nicht erreicht wird.

Beispiel 2

Reinigung einer hochkationischen Kartoffelstärke mit einem Substitutionsgrad von DS = 0,5

100g einer lufttrockenen kationischen Kartoffelstärke mit einem Substitutionsgrad von 0,5 (Typ PC-KS5000, 21,3% Feuchte ) werden in 200 g 75%igem Isopropanol eingerührt und durch Zugabe von 10,2 ml 10%iger Salzsäure auf einen pH-Wert von 6,0 eingestellt. Nach 20 minütigem Rühren wird die Suspension über eine Nutsche filtriert. Die nutschenfeuchte Stärke wird nochmals mit 162 g 75%igem Isopropanol 20 Minuten gerührt und abermals filtriert, wobei sich ein neutraler pH-Wert einstellt (ca. pH 7). Der Filterkuchen wird zweimal mit 50g 100%igem Isopropanol gewaschen, abfiltriert und anschließend im Vakuumtrockenschrank für 60 Minuten bei 80°C getrocknet.

Analysenergebnisse :

| Analyse | Ausgangsprodukt (alkalisch, ausreagiert.) | Endprodukt (gewaschen, neutralisiert) |
|---|---|---|
| Kationisches Polymer ( g atro ) | 78,7 | 76,3 |
| Feuchte ( % ) | 21,3 | 0,0 |
| pH-Wert ( 1%iger Kleister ) | 10,3 | 6,8 |
| Stickstoff-Gehalt ( % ) bez. auf atro Polymer | - | 3,04 |
| Brookfield-Viskosität ( mPa.s ) 1% atro Kleister | 160 | 208 |
| Theoretischer Substitutionsgrad | 0,60 | - |
| Praktischer Substitutionsgrad, DS | - | 0,525 |

Beispiel 3

Herstellung hochkationischer Kartoffelstärken mit einem Substitutionsgrad von 0,50 unter Verwendung verschiedener Aktivatoren
(Kationisierung in 3 Stufen)

Es wird wie in Beispiel 1 verfahren, nur mit dem Unterschied, daß lediglich dreimal mit jeweils einer Reagenzmenge, die einem theoretischen DS von 0,2 entspricht, kationisiert wird (theoreth. DS = 3 x 0,2 = 0,6). Außerdem werden neben dem Aktivator PC-3 auch die Aktivatoren PC-4 und PC-5 eingesetzt, wobei die Zugabemengen je Stufe 1,5 % für PC-3, 2,0 % für PC-4 und 2,5 % für PC-5, bezogen auf atro Stärke, betragen.

Analyse: 2,94 % Stickstoff (gewaschen, atro)
Substitutionsgrad (DS) = 0,50 ⇒ Ausbeute = 83,3 %

Mit der Verwendung der Aktivatoren PC-3, PC-4 und PC-5 können unterschiedliche Viskositäten der Stärkekleister bei konstantem Substitutionsgrad erzielt werden, wie aus der nachfolgenden Tabelle hervorgeht:

| Stärketype | prakt DS | verwendeter Aktivator | Brookfield-Viskosität (1000 UpM) | |
|---|---|---|---|---|
| | | | 1 %iger Kleister (20 °C) | 2 %iger Kleister(20 °C) |
| Kartoffelstärke | 0,5 | PC-3 | 120 mPas | 270 mPas |
| Kartoffelstärke | 0,5 | PC-4 | 240 mPas | 460 mPas |

(fortgesetzt)

| Stärketype | prakt DS | verwendeter Aktivator | Brookfield-Viskosität (1000 UpM) | |
|---|---|---|---|---|
| | | | 1 %iger Kleister (20 °C) | 2 %iger Kleister(20 °C) |
| Kartoffelstärke | 0,5 | PC-5 | 840 mPas | 1950 mPas |

Beispiel 4

Einsatz der hochkationisierten, natürlichen Polymere als Flockungshilfsmittel bei der Behandlung von Deponiesickerwasser

Zur Beurteilung der erfindungsgemäß hergestellten sehr hoch kationisierten Stärken als Flockungshilfsmittel wurde das Deponiesickerwasser einer Mülldeponie mit einem Feststoffgehalt von ca. 0,40 g/l und einem pH-Wert von 8,4 herangezogen. Es wurde wie folgt verfahren:

In 200 ml-Bechergläsern wurden jeweils 100 ml Abwasser vorgelegt. Anschließend wurden unter Rühren (200 UpM) verschiedene erfindungsgemäß hergestellte kationische Kartoffelstärken als 0,10 gew.%ige Lösungen zugegeben und weitere 10 Minuten bei 200 UpM gerührt. Nach einer Sedimentationszeit von insgesamt 90 Minuten wurden die am Boden der Bechergläser durch Flockung erhaltenen Sedimentationsvolumina als ml Feststoff gemessen. Anschließend wurde von jedem Becherglas von oben 50ml des behandelten Abwassers mittels Pipette entnommen. Diese oberen Abwasserfraktionen sowie die zugehörigen unteren Abwasserfraktionen wurden jeweils über vorher ausgeheizte (2h/120 °C) und gewogene Glasfaser- Rundfilter der Fa. Schleicher & Schuell (Ref.Nr.370005 ) filtriert. Die Filter wurden anschließend 2 h bei 120°C getrocknet und zurückgewogen. Aus den Wägungsdifferenzen wurde jeweils der Feststoffgehalt der beiden Abwasserfaktionen berechnet und in g / Liter ausgedrückt.

Die für die Flockung benötigten Polymer-Zusatzmengen bezogen auf 100g Abwasser sind:

| | |
|---|---|
| 50 g / t | = 5,0 ml 0,1%ige Polymerlösung |
| 30 g / t | = 3,0 ml 0,1%ige Polymerlösung |
| 15 g / t | = 1,5 ml 0,1%ige Polymerlösung |
| 10 g / t | = 1,0 ml 0,1%ige Polymerlösung |
| 5 g / t | = 0,5 ml 0,1%ige Polymerlösung |

Die Ergebnisse der Flockungsversuche sind in der Tab.1 gezeigt.

Die Versuche 1-9 zeigen, daß nur die erfindungsgemäß hergestellten Polymere mit einem praktischen Substitutionsgrad von 0,50 zu eine vollständigen Ausflockung der im Abwasser befindlichen Feststoffanteile führen. Der nach der Flockung bestimmte Gesamtfeststoffgehalt, welcher über beide Abwasserfraktionen gemittelt ist, liegt beim Einsatz der Polymere mit DS = 0,5 bei ca. 0,4 g/l, während bei den kationischen Stärken mit DS < 0,3 deutlich niedrigere Werte gefunden werden.

Beim Einsatz von kationischen Stärken mit Substitutionsgraden < 0,32 ist auch bei höheren Zugabemengen keine vollständige Ausflockung der Feststoffanteile mehr zu erreichen.

Die Versuche 8 und 9 zeigen, daß die Flockengröße und die Sedimentationsgeschwindigkeit durch die Verwendung unterschiedlicher Aktivator-Typen beeinflußt werden kann.

Die Ergebnisse machen deutlich, daß die erfindungsgemäß hergestellten Stärken mit Substitutionsgraden (DS) größer 0,3 gegenüber niedriger kationisierten Stärken deutliche Vorteile beim Einsatz als Flockungshilfsmittel besitzen.

Tabelle 1:

Verwendung hochkationisierter Stärke bei der Flockung von Feststoffen aus Deponiesickerwasser

Zeit: 90 min

Versuche 3-7: (PC-3)-Aktivator
Versuch 8: (PC-4)-Aktivator
Versuch 9: (PC-5)-Aktivator

| Versuchs-nummer | Substitutions-grad (DS) | Polymer-Zusatz g/t-Abwasser | Sedimentation | Gesamtfeststoff-gehalt (g/l) |
|---|---|---|---|---|
| 1 | ohne Zusatz | - | sehr langsame Flockung | 0,17 |
| 2 | native Stärke | 50 | sehr langsame Flockung | 0,18 |
| 3 | 0,04 | 50 | sehr langsame Flockung | 0,23 |
| 4 | 0,18 | 50 | schnelle Flockenbildung | 0,26 |
| 5 | 0,5 | 50 | große Flocken | 0,4 |
| 6 | 0,5 | 15 | langsame Sedimentation | 0,4 |
| 7 | 0,5 | 30 | große Flocken, schnelle Sedimentation | 0,42 |
| 8 | 0,5 | 30 | feine Flocken, langsame Sedimentation | 0,41 |
| 9 | 0,5 | 30 | sehr feine Flocken, langsame Sedimentation | 0,40 |

## Patentansprüche

1. Pulverförmige,in nicht vorverkleisterter Form vorliegende, substituierte natürliche Polymere, die über Ether- oder Iminogruppen gebundene Aminverbindungen der allgemeinen Formel

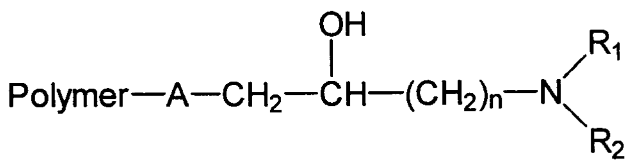

(I)

oder

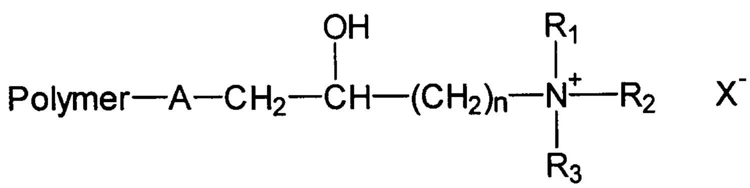

(II)

in der n eine ganze Zahl von 1 bis 3 ist, $R_1$,$R_2$ und $R_3$ gleiche oder verschiedene Alkylreste mit 1 bis 20 Kohlenstoffatomen oder $R_1$ den Benzylrest darstellt, A - O- und/oder -NH- bedeutet und $X^-$ Chlorid, Bromid, Sulfat, Nitrat oder Acetat bedeuten, insbesondere aber Chlorid, enthalten, wobei der Substitutionsgrad (D.S.) bei >0,32 liegt.

2. Substituierte Polymere gemäß Anspruch 1, **dadurch gekennzeichnet,** daß sie 0,02 bis 2,0 Gew.-% einer feinteiligen, hydrophoben oder hydrophilen Kieselsäure enthalten.

3. Substituierte Polymere gemäß den Ansprüchen 1 oder 2, **dadurch gekennzeichnet,** daß der pH-Wert der substituierten Polymeren bei > 8 (1 Gew.-%iger wässriger Kleister) liegt.

4. Substituierte Polymere gemäß den Ansprüchen 1 oder 2, **dadurch gekennzeichnet,** daß der pH-Wert der substituierten Polymeren bei 5-7 (1 Gew.-%iger wässriger Kleister) liegt.

5. Substituierte Polymere gemäß den Ansprüchen 1 bis 4, **dadurch gekennzeichnet,** daß es sich um substituierte Stärke handelt.

6. Substituierte Polymere gemäß den Ansprüchen 1 bis 4, **dadurch gekennzeichnet,** daß es sich um substituierte Cellulose handelt.

7. Substituierte Polymere gemäß den Ansprüchen 1 bis 4, **dadurch gekennzeichnet,** daß es sich um substituierte Galaktomannane handelt.

8. Verfahren zur Herstellung von substituierten natürlichen Polymeren mit einem Substitutionsgrad >0,32, gemäß den Ansprüchen 1 bis 7, dadurch gekennzeichnet, daß man die benötigte Gesamtmenge der Alkylenepoxide der allgemeinen Formeln

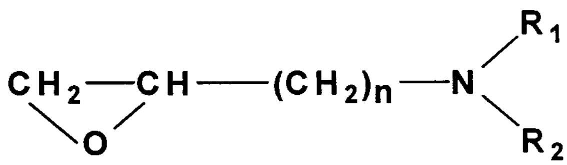

oder

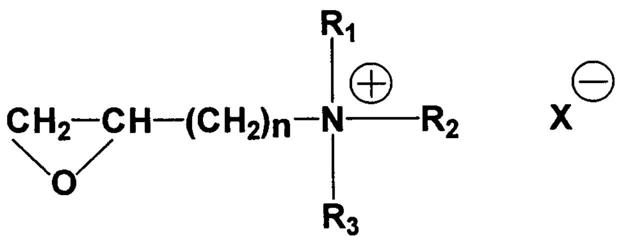

in denen n, $R_1$, $R_2$, $R_3$ dieselbe Bedeutung wie oben haben, in mindestens zwei Teilmengen aufteilt und diese in Gegenwart einer alkalisch wirkenden anorganischen Verbindung, 10 bis 35 Gew.-% Wasser (bezogen auf atro Stärke) und 0,02 bis 2 Gew.-% einer feinteiligen hydrophoben oder hydrophilen Kieselsäure (bezogen auf das Reaktionsgemisch) mit dem Polymeren stufenweise umsetzt.

**9.** Verfahren gemäß Anspruch 8,
**dadurch gekennzeichnet,**
daß man pro Stufe je Mol Polymer (gerechnet als Anhydroglucoseeinheit), 0,1 bis 0,3 Mol der Alkylenepoxide einsetzt.

**10.** Verfahren gemäß den Ansprüchen 8 und 9,
**dadurch gekennzeichnet,**
daß man statt der Epoxide die entsprechenden Chlorhydrine einsetzt.

**11.** Verfahren gemäß einem oder mehreren der vorhergehenden Ansprüche,
**dadurch gekennzeichnet,**
daß man die alkalisch wirkende anorganische Verbindung in Form einer pulverförmigen Mischung mit feinteiliger Kieselsäure einsetzt.

**12.** Verfahren gemäß einem oder mehreren der vorhergehenden Ansprüche,
**dadurch gekennzeichnet,**
daß man die alkalisch wirkende anorganische Verbindung oder deren Mischung mit einer feinteiligen Kieselsäure dem zu substituierenden Polymer vor den Alkylenepoxiden gemäß den Formeln (III) oder (IV) zusetzt, wobei man die alkalisch wirkende Verbindung gegebenenfalls in flüssiger Form einsetzt.

**13.** Verfahren gemäß einem oder mehreren der vorhergehenden Ansprüche,
**dadurch gekennzeichnet,**
daß man das Gemisch aus Polymer, alkalisch wirkender Verbindung, gegebenenfalls feinteiliger Kieselsäure und Alkylenepoxiden in einem Intensivmischer homogenisiert, dann ausschleust und in dem vorgesehenen Lagerbehältnis ausreagieren läßt.

**14.** Verfahren gemäß 13,
**dadurch gekennzeichnet,**
daß man einen Pflugscharmischer verwendet.

**15.** Verfahren gemäß 13,
**dadurch gekennzeichnet,**
daß man einen Durchflußbefeuchtungsmischer verwendet.

**16.** Verfahren gemäß einem oder mehreren der vorhergehenden Ansprüche,
**dadurch gekennzeichnet,**
daß man dem Polymer während oder nach der Umsetzung eine Säure zusetzt.

**17.** Verwendung der kationischen Polymere gemäß Anspruch 1 als Flockungshilfsmittel.

**18.** Verwendung der kationischen Polymere gemäß Anspruch 1 als Schlammentwässerungsmittel, Verdickungsmittel, Retentionshilfsmittel und Störstoff-Fänger bei der Papierherstellung.

**19.** Verfahren gemäß den Ansprüchen 8 bis 16,
**dadurch gekennzeichnet,**
daß man die so erhaltenen substituierten Polymere durch Waschen reinigt.

**20.** Verwendung der gereinigten Poymere gemäß Anspruch 19 als Zusatzstoffe in der Kosmetik- und Pharmaindustrie.